# EUROPEAN PATENT APPLICATION

(11) **EP 1 801 230 A1**
(43) Date of publication of application: **27.06.2007**
(21) Application number: 05781376.8
(22) Date of filing: 02.09.2005
(51) Int. Cl.: C12Q 1/02, C12N 5/06, C12N 15/09, G01N 33/53, G01N 33/58, G01N 33/68

(54) **METHOD OF SELECTING PROTEIN EASILY UNDERGOING ANTIGEN-PRESENTATION BY DENTRITIC CELLS**

(30) Priority: 03.09.2004 JP 2004257775
(71) Applicant: Medical and Biological Laboratories Co., Ltd., Nagoya-shi, Aichi 460-0002 (JP); Keio University, Tokyo 108-8345 (JP)
(72) Inventor: IMAI, Jun, Tokyo 2010003 (JP); MARUYA, Mikako, Tokyo 1710031 (JP); HASEGAWA, Hironori, Nagano 3960011 (JP); KOYASU, Shigeo, Tokyo 1770042 (JP); YAHARA, Ichiro, Yokohama-shi, Kanagawa 2220012 (JP)
(74) Representative: Denison, Christopher Marcus
(86) International application number: PCT/JP2005/016110
(87) International publication number: WO 2006/025525

(57) **Abstract**

The present inventors worked on elucidating the mechanism of antigen cross-presentation by dendritic cells, and revealed that foreign antigens taken up in dendritic cells are degraded by proteasomes after undergoing polyubiquitination. They discovered a positive correlation between the uptake amount of an antigenic protein by dendritic cells or the level of polyubiquitination and the level of antigen presentation of the antigenic protein. Based on these findings, methods were developed for predicting the intensity of cross-presentation of an antigenic protein by measuring the amount of antigenic protein taken up or polyubiquitinated protein. The use of these methods allows selecting antigenic proteins that are readily presented as antigens from among a number of proteins. The methods of the present invention enable suitable cancer immune vaccines to be provided through selection of more suitable cancer-specific proteins.

## Description

### Technical Field

The present invention relates to methods for selecting proteins that are readily presented as antigens on dendritic cells, wherein the methods are based on cross-presentation mechanisms by dendritic cells.

### Background Art

When cells in a living body undergo cancerization, normally, immune defense mechanisms function to remove the cancerized cells. Cancer is thought to develop when the cancerized cells escape the surveillance of the immune defense mechanisms. The cancerized cells are mainly removed through cellular immunity.

If a cellular immune mechanism is activated by a suitable procedure, it can act to strongly attack and remove cancers that have formed. Therefore, activation of cellular immunity may become a method for efficient removal of cancer cells. Cancer immunotherapy has already drawn attention as a means to treat cancer. Various methods have been researched/developed so far, such as cell introduction therapies in which LAK cells, CTL cells, and such are administered, and vaccine therapies in which dendritic cells or peptides are administered as vaccines. However, activation of cellular immunity cannot be separated from induction of immunological tolerance. Therefore, how to achieve an activation that is efficient and specific to the target is a major issue in tumor immunity.

Dendritic cell vaccine therapies are methods for treating cancers by harvesting dendritic cells from patients, making those dendritic cells phagocytose cancer antigen proteins *ex vivo,* returning the dendritic cells now presenting antigenic peptides into the body of the cancer patients, and specifically enhancing immunity against cancer. However, for the reason that effective cancer antigens differ depending on the patient, and so on, dendritic cell vaccine therapies are not necessarily effective therapeutic methods for all patients. If it were possible to pre-select cancer antigen proteins that can be effectively presented as antigens on dendritic cells, the efficacy ratio of dendritic cell vaccine therapy might also increase. However, at present there is no practical evaluation system for assessing the effectiveness of cancer antigen proteins.

As an experimental evaluation system, a system for evaluating antigen presentation level using DC2.4 cells and naive CD8⁺ T cells from OT-I mice has been constructed. DC2.4 cells are a mouse cell line whose MHC class I haplotype is H-2Kb. OT-I (H-2Kb) mice are transgenic mice expressing T cell receptors (TCRα and β) that recognize an OVA-derived antigenic peptide (SIINFEKL) (SEQ ID NO: 15) presented on the H-2Kb molecule. Therefore, the CD8⁺ cells of OT-I mice recognize the OVA peptide presented by DC2.4 cells and are then activated. The amount of IL-2 produced by the activated OT-I CD8⁺ T cells serves as an index of T cell stimulation by OVA antigens presented by DC2.4 cells. However, this evaluation system requires animals that express TCRs specific to particular antigens. Therefore, for antigenic proteins in general, constructing an evaluation system similar to the above evaluation system has to be considered completely impossible.

An understanding of the immune system is necessary to provide immunotherapy with higher efficacy. Cellular immunity is a type of acquired immunity. Acquired immunity is classified into two types: humoral immunity and cellular immunity. Humoral immunity is an immune reaction mediated by antibodies against infection by bacteria, parasites, and such, during which B cells, which produce antibodies, are mainly activated. On the other hand, cellular immunity is a reaction that directly destroys cancerized cells and virus-infected cells, during which CD8⁺ cytotoxic T cells are activated.

T cells use T cell receptors (TcRs) expressed on their cell membrane to recognize a group of protein complexes called major histocompatibility complexes (MHCs) on other cells. MHC molecules encoded by MHC genes form complexes with oligopeptides produced by the degradation of proteins in cells, and are then expressed on cell surfaces.

There are two types of MHC molecules: Class I and Class II. MHC II molecules comprise an α chain and a β chain, are only expressed in antigen-presenting cells, mainly present peptides derived from antigens taken up from outside the cell (infectious pathogens and such) to helper T cells, and play an essential role in the expression of humoral immunity.

In contrast, MHC I molecules, which comprise a main chain and subchain (β2 microglobulin), are expressed on the cell membrane of almost all nucleated cells. Generally, MHC I molecules are bound to oligopeptides derived from the degradation products of proteins in their own cell, and are self markers in cellular immunity. With regards to discriminating between self and non-self, MHC Class I molecules presenting self-protein-derived peptides are recognized as self, and a cellular immune reaction is not induced. On the other hand, when MHC Class I molecules are presenting peptides derived from non-self proteins (for example, cancerized cells, virus-infected cells, transplanted organs, or such), a cellular immune reaction is induced.

Activation of cellular immunity requires the presentation on an MHC I molecule of a non-self-derived peptide along with a costimulatory factor to CD8 antigen-positive T cells. If this activation does not occur efficiently, CD8⁺ T cells that recognize non-self can not differentiate into activated cells (CTLs) exhibiting cytotoxicity against cells presenting non-self antigens, even though they may be present.

Dendritic cells, along with macrophages and B cells, are referred to as professional antigen-presenting cells, and play a notably important and special role in the presentation of foreign antigens. Dendritic cells take up carcasses or fragments of cancerized cells or virus-infected cells and present proteins included in these on MHC class I molecules. Therefore, in macrophages and B cells, foreign antigens are presented on MHC class II molecules, whereas in dendritic cells, foreign antigens are presented on MHC class I as well as MHC class II molecules. This phenomenon is referred to as antigen cross-presentation and is characteristic of dendritic cells. Cross-presentation of foreign antigens by dendritic cells plays a critically important role in the removal of infected cells and cancer cells.

Foreign antigenic proteins taken up by dendritic cells are transported and degraded, then peptides derived from the antigenic proteins form complexes with MHC class I molecules, and these are expressed on cell membranes; however, this series of mechanisms is only partly elucidated. So far, the cellular mechanism of cross-presentation by dendritic cells is described as follows: first, antigenic proteins derived from cancer cells or virus-infected cells are taken up into the lumen of dendritic cells (a compartment surrounded by a membrane) by endocytosis (which also comprises phagocytosis, uptake of immune complexes, and such). Antigenic proteins that have been taken up, or their degradation products, translocate to the cytoplasm by passing through the membrane *via* an unknown mechanism. They are then degraded by proteasomes, in the same way as for endogenous antigenic proteins. Peptides which are the degradation products are transported to the ER by TAP transporters, then form complexes with MHC I molecules in the ER, and are expressed on the cell membrane *via* an ordinary pathway. However, the kind of intracellular compartments the antigenic proteins are taken into from the outside, the way the antigenic proteins taken up or their degradation products pass through the membrane to translocate to the cytoplasm, and such have not been solved yet

### Disclosure of the Invention

### Problems to be Solved by the Invention

The present invention was achieved in view of the above circumstances. An objective of the present invention is to elucidate the mechanism of antigen cross-presentation by dendritic cells, and to provide methods for selecting antigenic proteins that are readily presented as antigens.

### Means to Solve the Problems

To solve the above problems, the present inventors worked on elucidating the mechanism of antigen cross-presentation by dendritic cells. There are two reactions key to elucidating the mechanism of antigen cross-presentation by dendritic cells: 1) the kind of intracellular compartments the antigenic proteins are taken into from the outside; and 2) the way in which the antigenic proteins taken up or their degradation products pass through the membrane to translocate to the cytoplasm.

To elucidate the above mechanism, cell fractionation and Western blotting were carried out, and the intracellular localization of the foreign antigens taken up as well as the changes taking place over time were observed. These results revealed for the first time that the foreign antigens taken up accumulate in membranous organelles in their undegraded form, and are then degraded *via* the ubiquitin-proteasome pathway. Furthermore, the present inventors realized that the above pathway is essentially the same as ER-associated degradation (ERAD), and hypothesized that that the foreign antigens accumulated in the dendritic cells are degraded by ERAD or by a very similar proteolytic mechanism.

The present inventors set out to prove this hypothesis using three methods. First, coimmunoprecipitation was performed to examine for any interaction between the accumulated foreign antigens and the various ER-associated proteins. In the second method, ERAD-inhibiting treatments were carried out on dendritic cells and whether the degradation of accumulated foreign antigens is affected was examined. Thirdly, RNAi methods were used to knock-down an ER-associated protein and the effect on the accumulated foreign antigens was examined.

The results of the above examinations revealed that in dendritic cell cross-presentation, foreign antigens are taken up into membrane vesicles, bind to endoplasmic reticulum molecular chaperones such as BiP and calreticulin, and undergo retrograde transport into the cytoplasm; that foreign antigens that transferred to the cytoplasm are polyubiquitinated in their undegraded form; that the ubiquitin E3 ligase involved in the above polyubiquitination is CHIP; and that the polyubiquitinated foreign antigens are degraded by proteasomes. These are all novel findings discovered for the first time by the present inventors, and all support the above hypothesis.

The present inventors also succeeded in measuring the antigenic proteins taken up in dendritic cells and the antigenic proteins polyubiquitinated after uptake, and discovered a positive correlation between the amount of antigenic proteins taken up by dendritic cells or the amount of polyubiquitinated antigenic proteins and the level of antigen presentation of the antigenic proteins. Based on these new findings, the present inventors developed methods that measure polyubiquitinated proteins or the amount of uptake of antigenic proteins, and predict the intensity of cross-presentation of these antigenic proteins. By using these methods to measure how readily a number of antigenic proteins are presented as antigens, the antigenic proteins that are readily presented as antigens can be selected from among these proteins. Therefore, the present invention relates to methods for selecting antigenic proteins that are readily presented as antigens. More specifically, it provides the following inventions:
(1) a method for selecting a protein that is readily presented as an antigen on a dendritic cell, wherein a step of contacting a protein to an isolated dendritic cell is followed by at least one or more of the following steps of:
   (a) measuring the amount of the protein taken up in the dendritic cell;
   (b) measuring the amount of the protein polyubiquitinated in the dendritic cell; and
   (c) measuring the amount of the protein taken up by the dendritic cell that remains in the dendritic cell;
(2) a method for producing a dendritic cell proficient at inducing a CTL, wherein the method comprises the steps of:
   contacting a protein to an isolated dendritic cell;
   measuring at least any one or more of the following amounts of protein:
      (a) the amount of the protein taken up in the dendritic cell;
      (b) the amount of the protein polyubiquitinated in the dendritic cell; and
      (c) the amount of the protein taken up by the dendritic cell that remains in the dendritic cell;
         selecting a dendritic cell that has taken up the protein, based on the measurements in the previous step; and
         collecting the dendritic cell selected in the selection step;
(3) the method of (1) or (2), wherein the protein is labeled and then used;
(4) the method of any one of (1) to (3), wherein the step of measuring the amount of protein polyubiquitinated in the dendritic cell comprises the use of an anti-ubiquitin antibody; and
(5) a method for testing capacity as a cancer antigen vaccine, which comprises the method of any one of (1), (3), and (4).

### Brief Description of the Drawings

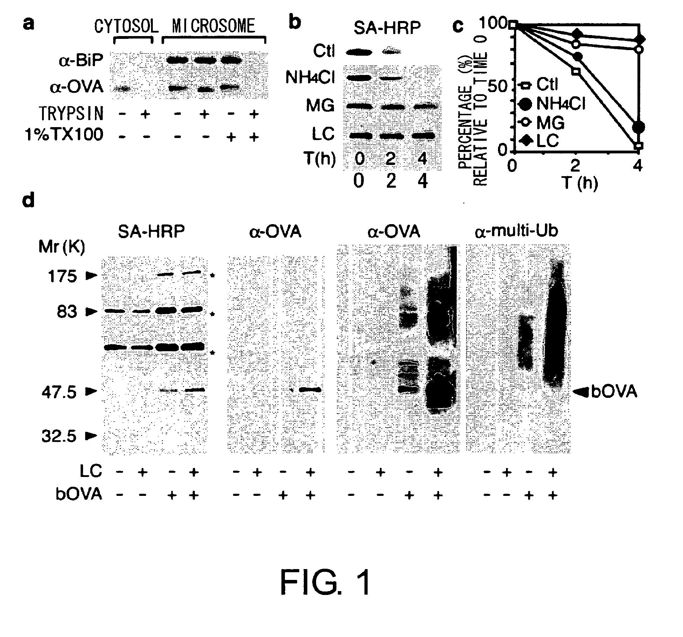

Fig. 1 shows the characteristics of OVA taken up by DC2.4 cells. (a) shows a Western blot of cytosol and microsomal fractions using various antibodies. Trypsin treatment of microsomal fractions did not affect accumulated OVA and BiP. Triton X-100 was used to lyse membranes. (b) shows the effect of proteasome inhibitors and lysosome inhibitors on bOVA accumulation. The proteasome inhibitors MG-132 (MG: 10 µM) and lactastatin (LC: 2 µM) reduced the degradation of bOVA taken up into cells, but NH₄Cl (50 mM) did not reduce this degradation. DC2.4 cells were pulsed with bOVA and chased. bOVA was recovered using SA beads, then separated by SDS-PAGE followed by Western blotting using SA-HRP Ctl: a control not containing inhibitors. (c) shows the results of quantifying that shown in (b). (d) shows that bOVA is polyubiquitinated before degradation. Cell-bound bOVA has the same molecular weight as intact OVA. Portions of bOVA were polyubiquitinated. Asterisks in the left panel indicate non-specific binding with SA-HRP.

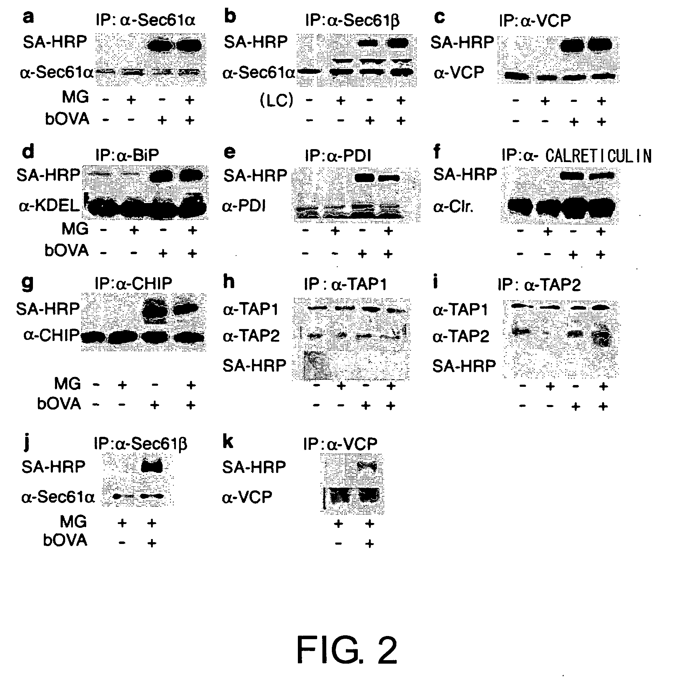

Fig. 2 shows that ERAD-related proteins interact with cell-bound OVA. (a) shows that Sec61α interacts with bOVA. DC2.4 cells were incubated with bOVA and subjected to co-immunoprecipitation with anti-Sec61α. bOVA was added at 2.5 mg mL⁻¹ to cell lysates before immunoprecipitation (the two left-hand lanes in each panel). After SDS-PAGE, blotting was performed using SA-HRP and anti-Sec61α antibody. (b) shows that Sec61β interacts with bOVA. (c) shows that VCP interacts with bOVA. (d) shows that BiP interacts with bOVA. (e) shows that PDI interacts with bOVA. (f) shows that calreticulin interacts with bOVA. (g) shows that CHIP interacts with bOVA. (h) shows that TAP1 does not interact with bOVA. (i) shows that TAP2 does not interact with bOVA. (j) shows that bOVA interacts with Sec61β even in BMDCs. (k) shows that bOVA interacts with VCP even in BMDCs.

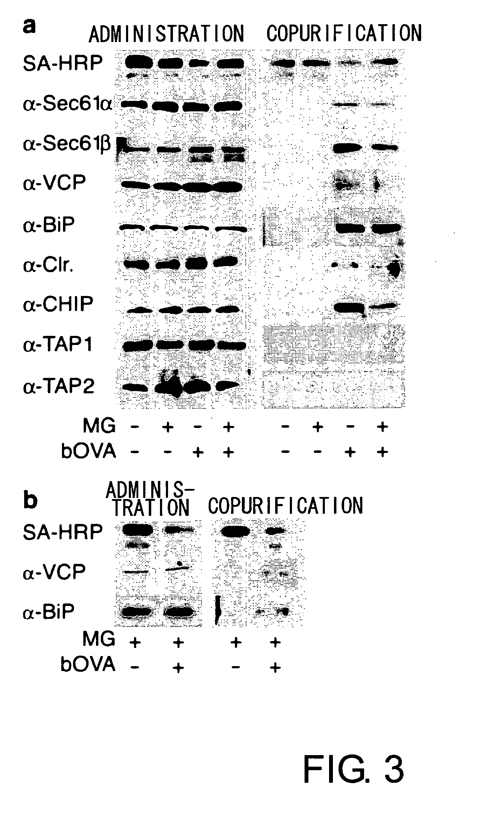

Fig. 3 shows that ERAD-related proteins are co-purified with bOVA taken up in cells. (a) shows co-purification with ERAD-related substances derived from DC2.4 cells. (b) is a figure showing the results of co-purification of VCP and BiP derived from BMDC cells.

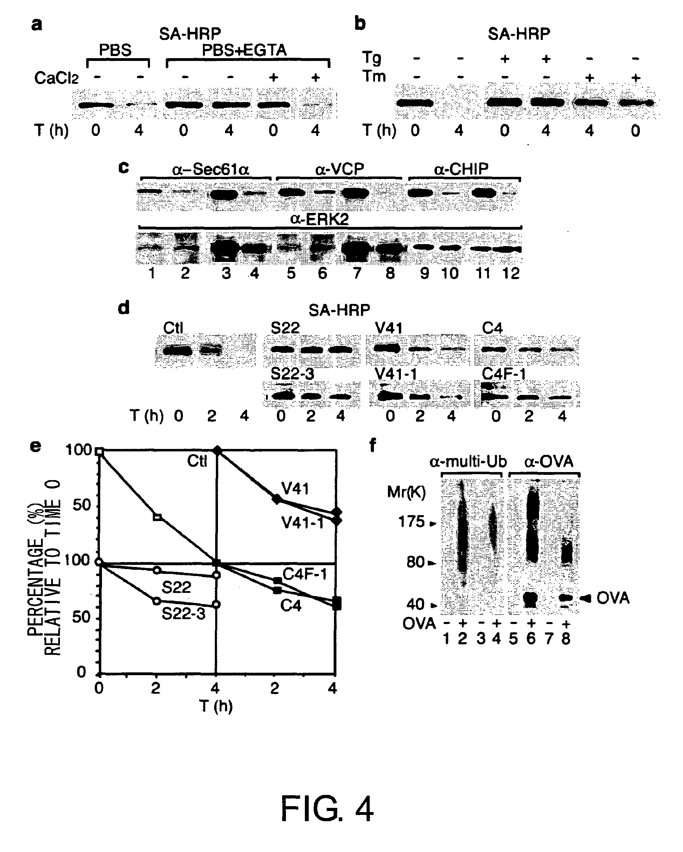

Fig. 4 shows that ERAD inhibition and knock-down of ERAD-related proteins by RNAi methods reduces bOVA degradation. (a) shows that bOVA degradation is inhibited by Ca²⁺ depletion, and recovers upon re-addition of Ca²⁺ (1mM). (b) shows that bOVA degradation is inhibited by thapsigargin (Tg: 300 nM) or tunicamycin (Tm: 2µg). (c) shows the effect of knocking-down ERAD-related proteins using RNAi methods. Uncloned stable transfectants (S22: Sec61-22; V41: VCP-41; C4: CHIP-4) and cloned knocked-down cell lines (S22-3: derived from S22; V41-1: derived from V41; and C4F-1: derived from C4) into which RNAi was transfected showed reduced expression of ERAD-related proteins (Lanes 3, 5, 7, 9, 10, and 11). The controls are lane 2: S22; lane 4: S22-3; lane 6: V41; lane 8: V41-1; lane 10: C4; and lane 12: C4F-1. (d) shows bOVA degradation when parental DC2.4 cells and knocked-down DC2.4 cells were separately incubated with bOVA. (e) is a diagram quantifying the results of (d) above. (f) shows that polyubiquitination of bOVA was reduced in C4. Lanes 1, 2, 5, and 6 are the controls, and lanes 3, 4, 7, and 8 are C4.

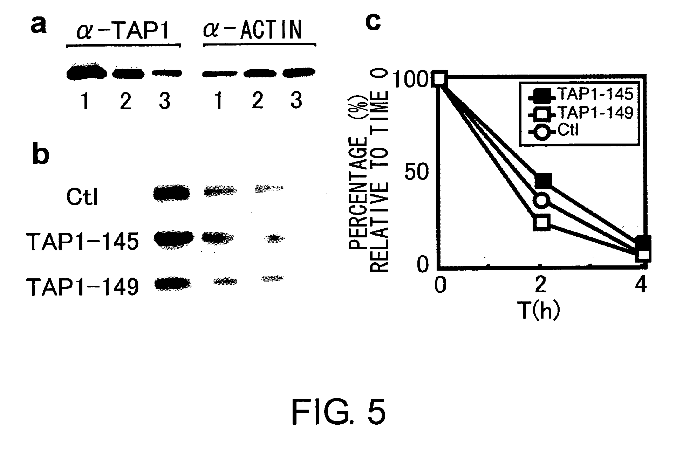

Fig. 5 shows that TAP knock-down by RNAi methods does not affect bOVA degradation. (a) Compared to parental DC2.4 cells (lane 1), the uncloned stable transfectants (lane 2: TAP1-145; lane 3: TAP1-149) showed a specific reduction in TAP-1 protein expression. (b) shows the results of measuring bOVA degradation after separately incubating parental DC2.4 cells and knocked-down DC2.4 cells with bOVA. From the left: zero hour, two hours, and four hours. (c) is a diagram quantifying the results of (b) above.

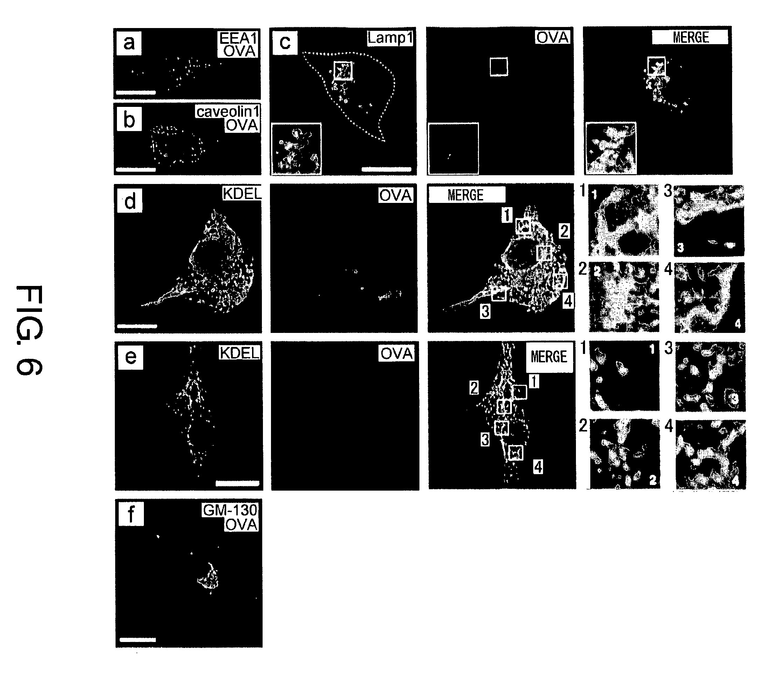

Fig. 6 shows the intracellular localization of exogenously added OVA in DC2.4 cells. The intracellular localization of OVA was detected by labeling with an anti-OVA antibody (red). Early endosomes (a), caveosomes (b), late endosomes (c), ER (d and e) and Golgi apparatus (f) are shown in green. In (c), the cell periphery is indicated by a dotted line. Insets in (c) and (d) show the area of the cell indicated by squares in a higher magnification.

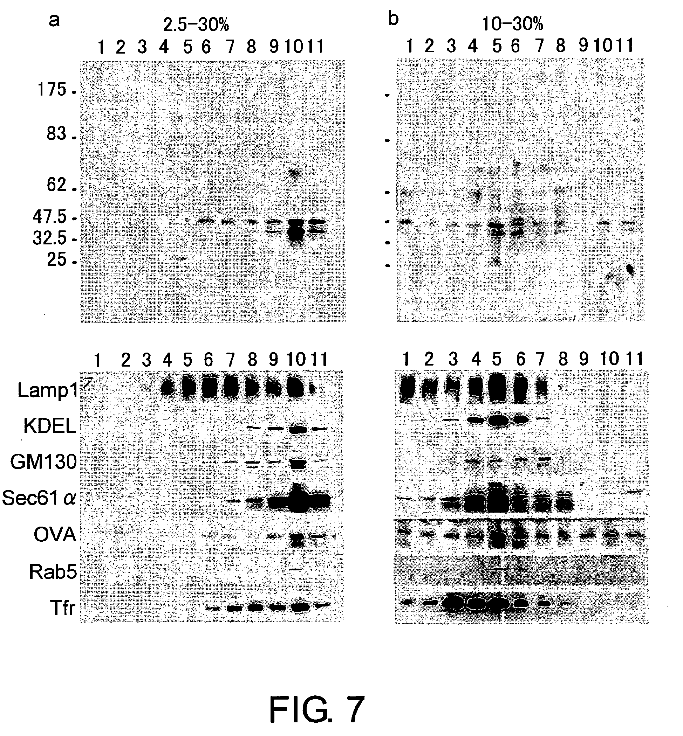

Fig. 7 shows the results of performing density gradient centrifugation on membrane fractions of DC2.4 cells. (a) DC2.4 cells incubated with OVA were homogenized, membrane fractions were prepared and subjected to 2.5-30% Optiprep density gradient centrifugation. The upper panel was obtained by separating each of the fractions by SDS-PAGE and then blotting using an anti-OVA antibody. The lower panel was obtained by blotting the same membrane sheet shown in the upper panel with various antibodies. (b) Membrane fractions were analyzed on a 10-30% density gradient. The lane numbers indicate the fractions of each density gradient.

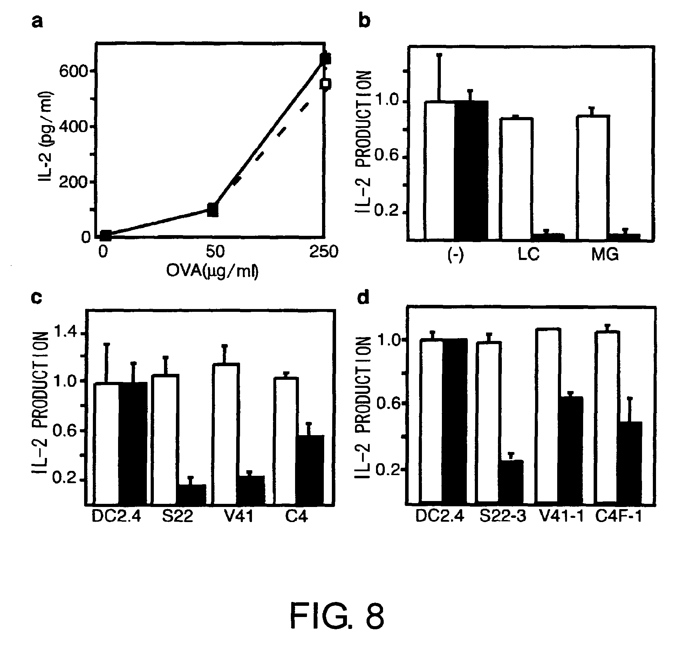

Fig. 8 shows the results of knocking down Sec61, VCP, and CHIP. Parental DC2.4 cells or knocked-down DC2.4 cells were separately incubated with SIINFEKL (SEQ ID NO: 15) peptide or OVA, and the stimulation of OT-I CD8⁺ T cells was observed using the IL-2 production level as an index. (a) is a dose-response curve showing the relationship between OVA and IL-2 production. White columns indicate OVA and black columns indicate bOVA. (b) Lactastatin (10 µM) and MG-132 (25 µM) suppressed stimulation by OVA (black bars) but did not suppress stimulation by the SIINFEKL (SEQ ID NO: 15) peptide (white bars). (c) A reduction in OVA stimulation was observed with DC2.4 cells in which Sec61 (S22), VCP (V41), or CHIP (C4) was knocked down. (d) Clones from knocked-down cells essentially showed the same results as in (c).

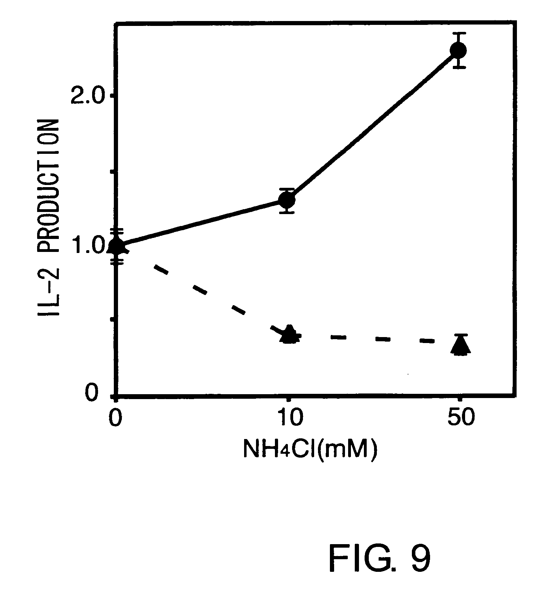

Fig. 9 shows the results of observing the effect of NH₄Cl on antigen presentation by MHC class and class II molecules on DC2.4 cells. DC2.4 cells pretreated in the presence of NH₄Cl were cultured with OVA, and then the stimulation of OT-I CD8⁺ T cells in response to MHC class antigen presentation (circles) and stimulation of OT-II CD4⁺ T cells in response to MHC class II antigen presentation (triangles) was determined by measuring IL-2 production levels. IL-2 production level in the presence of NH₄Cl was expressed as a ratio relative to the control (IL-2 production was 700 pg/mL relative to OT-I, and 20 pg/mL relative to OT-II).

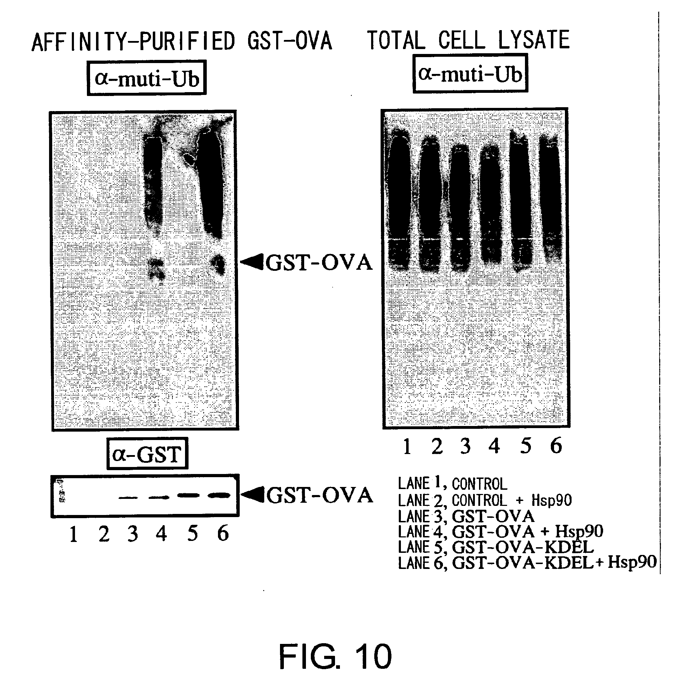

Fig.10 shows the result of incubating GST-OVA or GST-OVA-KDEL with DC2.4 cells in the presence or absence of HSP90, collecting GST-OVA from the cells, and then performing SDS-PAGE and Western blotting. Lanes 1 and 2 show the results from the cells alone, and lanes 3 and 4 show the results of incubating the cells with GST-OVA in the presence or absence of HSP90. Regarding lane 4 and its control (lane 3), a comparison of the amount of GST-OVA taken up (lower photograph) and the amount of polyubiquitinated GST-OVA shows that the increase in the amount taken up in the presence of HSP90 is not great, but that the increase in polyubiquitination in the presence of HSP90 is very pronounced. When lane 6 and lane 4 are compared, the amount of polyubiquitinated GST-OVA in lane 6 is significantly larger. This revealed that when HSP90 is present, the addition of KDEL (SEQ ID NO: 3) increases the uptake of GST-OVA and promotes polyubiquitination.

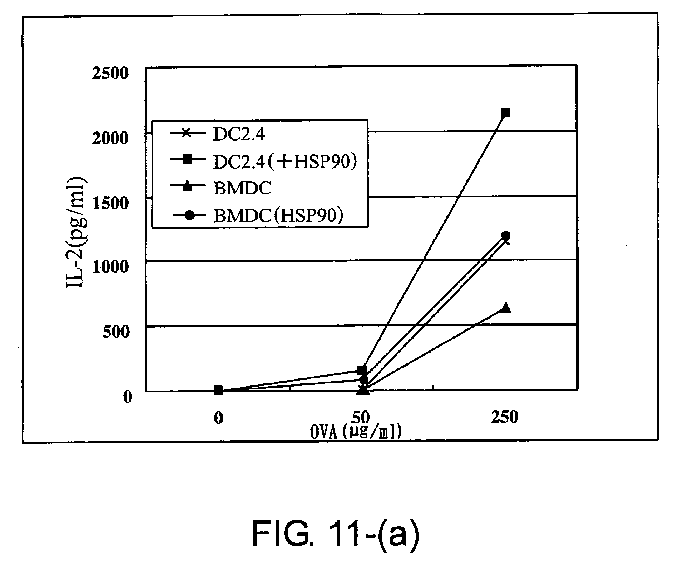

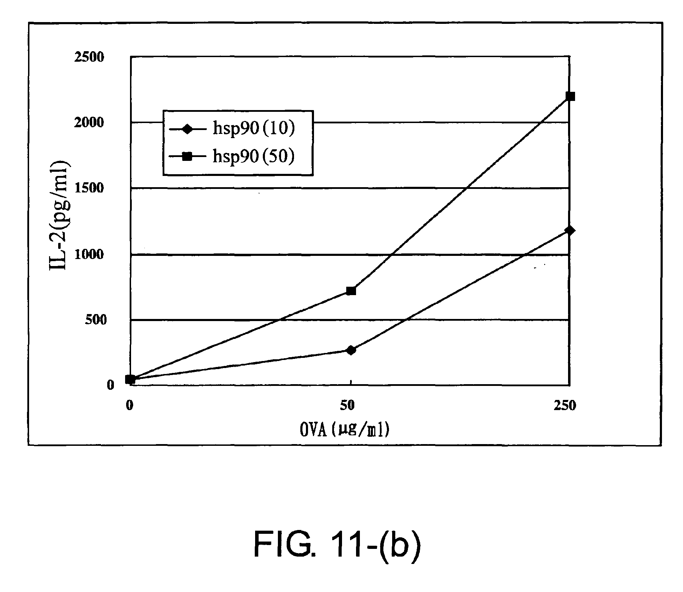

Fig. 11-(a) shows the results of incubating DC2.4 cells or BMDCs with OVA in the presence or absence of HSP90, and then measuring antigen presentation to OT-1 CD8⁺ cells. Addition of HSP90 was shown to increase antigen presentation. Fig. 11-(b) shows the results of measurements under the same conditions as for the data for DC2.4 cells in (a) above, but with different concentrations of Hsp90 (10 µg/mL and 50 µg/mL). This shows that antigen-presenting ability increases in a manner dependent on Hsp90 concentration.

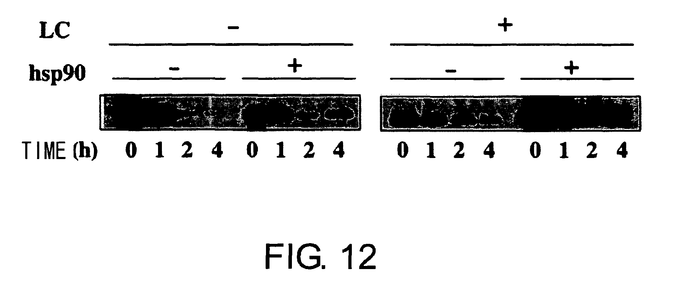

Fig. 12 shows the result of incubating DC2.4 cells with OVA in the presence or absence of lactacystin (LC), and in the presence or absence of Hsp90, and then measuring the OVA taken up into the cells. The time refers to incubation time. As the eight right-hand lanes show, uptake of OVA increased when Hsp90 was present.

### Best Mode for Carrying Out the Invention

The present invention relates to methods for selecting proteins that are readily presented as antigens by dendritic cells. The present invention is methods for selecting proteins that are readily presented as antigens, in other words, proteins proficient at inducing CTLs, or proteins with high immunity-inducing effects. Since methods for directly and generically evaluating the immunity-inducing effects of proteins do not exist, as an alternative, the present methods divide the series of processes of cross-presentation by dendritic cells into three stages, quantify each stage, and then carry out a relative evaluation of the ease of protein presentation as an antigen based on these quantified values for each stage.

The methods of the present invention comprise at least any one or more of the steps of "contacting a test protein with isolated dendritic cells, and measuring the amount of test protein taken up in the dendritic cells" (hereinafter, abbreviated as the "step of measuring the amount taken up"), "contacting a test protein with isolated dendritic cells, and measuring the amount of test protein polyubiquitinated in the dendritic cells" (hereinafter, abbreviated as the "step of measuring the polyubiquitination level"), and "contacting a test protein with isolated dendritic cells, and measuring the residual amount of test protein taken up in the dendritic cells" (hereinafter, abbreviated as the "step of measuring the residual amount"). The methods must only include at least one of these steps, or they may include a combination of any two of these steps, or all three of these steps.

Test proteins in the methods of the present invention may be of any type or size so long as they can become subjects of phagocytosis by dendritic cells, and subjects of proteasome degradation. Generally, molecules composed of polypeptide chains are often differentiated by referring those with a molecular weight of 5000 or more as proteins, and those with a molecular weight of 5000 or less as peptides (Encyclopedic Dictionary of Chemistry, Tokyo Kagaku Dojin); however, so long as they are taken up by dendritic cells and undergo proteasome degradation, they are comprised in the test proteins of the present invention, without limitation on their molecular weight. Additionally, they may be simple proteins, or conjugated proteins such as nuclear proteins, lipoproteins, glycoproteins, chromoproteins, or metalloproteins. Cancer antigen proteins and infected virus-derived proteins are suitable subjects in the present methods.

The dendritic cells used in the methods of the present invention are preferably mammal-derived cells, and more preferably human-derived cells. Cell lines may be used instead of isolated cells. When selecting proteins for the purpose of performing tailor-made therapies, the use of cells derived from the patient to be treated is preferable.

The dendritic cells used in the methods of the present invention are preferably immature cells. This is because immature dendritic cells take up antigens from outside very well, and as they mature, they more strongly present the antigens that had been taken up. Such dendritic cells can be prepared by methods for using GM-CSF to induce them from CD34-positive progenitor cells in bone marrow or peripheral blood or from monocytes, or by methods for directly separating them from peripheral blood mononuclear cells, and other such methods.

In order for a foreign antigenic protein-derived peptide to be presented as an antigen on the surface of dendritic cells by cross-presentation, first, the foreign antigenic protein must be taken up in the dendritic cells. The step of measuring the amount taken up is a step of measuring the amount of a test protein taken up in dendritic cells. Basically, for this step it is sufficient to make a dendritic cell take up a test protein, and measure the protein taken up in the cell.

To make dendritic cells take up a test protein, the step of contacting the test protein with dendritic cells can be carried out. Dendritic cells. contacted with a test protein take up the protein into the cells by phagocytosis or endocytosis. To prevent degradation by proteasomes of the protein taken up at this stage, contact between the dendritic cells and the test protein may be carried out in the presence of proteasome inhibitors such as lactastatin or MG-132.

Dendritic cells that have taken up the protein can be used as samples for measurement by preparing microsomal fractions by centrifugation. Microsomal fractions are used because when an antigenic protein is taken up by dendritic cells, it accumulates within membrane vesicles comprising the endoplasmic reticulum or late endosomes. Alternatively, measurement samples prepared by thoroughly washing the cells and then preparing cell extracts may also be used.

The amount of test protein taken up in dendritic cells can be measured using an antibody against the test protein. Alternatively, the test protein can be pre-labeled, and measurements can be made using the label. Labeling can be carried out, for example, by using biotin, histidine, GST, radioactive labeling materials, or fluorescence labeling materials such as FITC, Cy3, Cy5, or GFP. Labeling materials that do not affect antigenicity are preferably selected.

As in the Example of the biotin-labeled OVA antigen, the measuring method can employ SDS-electrophoresis of the sample, followed by Western blotting using streptavidin, and then quantification using image analysis. Alternatively, the proteins in the samples may also be measured by various known immunoassays such as EIA, ELISA, RIA, CLIA, and CLEIA methods.

Measured values for the amounts of each test protein are obtained by performing as above. Those test proteins found to have higher measured values are proteins that are relatively easily taken up by dendritic cells among the test proteins.

This is explained using an example. First, a number of test proteins are prepared. The test proteins are labeled with biotin. Next, to make dendritic cells take up the labeled proteins, the dendritic cells and the labeled proteins are incubated for a given period of time. After SDS electrophoresis, Western blotting using streptavidin is carried out, and the proteins are quantified.

The step of measuring the polyubiquitination level is a step of measuring the amount of polyubiquitinated protein from among the foreign antigenic proteins taken up in dendritic cells. This step is based on the present inventors' finding that polyubiquitination of a foreign antigenic protein is a prerequisite for its degradation by proteasomes during the process of cross-presentation by dendritic cells. As shown in the Examples, a positive correlation between the measured value for a polyubiquitinated protein and the level of antigen presentation of that protein was confirmed. Therefore, measurement of the polyubiquitination level may serve as one index for knowing the intensity of antigen presentation.

The step of measuring the polyubiquitination level can be carried out using both an anti-polyubiquitin antibody and an antibody against the test protein or a label-binding substance if the protein is labeled.

As in the Examples, one example of this is to incubate biotin-labeled test proteins with dendritic cells in the presence of proteasome inhibitors, produce cell extracts, and then carry out Western blotting using streptavidin and an anti-polyubiquitin antibody to quantify the amount of polyubiquitinated antigenic proteins. An alternative method may be purification using an affinity column that uses an anti-test protein antibody or a label-binding substance, then the use of various immunoassays that use an anti-polyubiquitin antibody to measure the amount of polyubiquitinated protein in the purified substance.

An objective of the step of measuring the residual amount is essentially to know the rate at which a polyubiquitinated foreign antigenic protein is degraded by proteasomes. In order for a foreign antigen to be presented as an antigen, it must be degraded by proteasomes in the dendritic cells. Therefore, the rate of degradation by proteasomes may serve as one index for knowing whether a foreign antigen is readily presented as an antigen. The rate of degradation by proteasomes can be determined by measuring over time the amount of polyubiquitinated foreign antigenic protein that remains without being degraded by proteasomes. The rate of decrease in the amount of undegraded protein reflects the rate of degradation by proteasomes.

Specifically, dendritic cells are contacted with test proteins in the absence of proteasome inhibitors, and the amount of test protein in the dendritic cells is measured over time. Similarly to the step of measuring the amount taken up, the measurement methods can be carried out by various known methods using anti-test protein antibodies or using labels. This is explained using an example, Labeled test proteins and dendritic cells are incubated in the absence of proteasome inhibitors. Samples are collected over time, starting from time 0. After washing the cells, an extract is produced, and SDS-electrophoresis and Western blotting are carried out to quantify the residual labeled proteins. The rate is calculated from the changes in the residual protein over time.

The measured values for each step obtained in this way serve as indexes of the ease with which test proteins are presented as antigens. Test proteins are more readily presented as antigens when their measured values are high for the step of measuring the amount taken up and for the step of measuring the polyubiquitination level, and when their rate of decrease in residual amount is high in the step of measuring the residual amount Comparison of the measured values for each of the test proteins allows evaluation as to which proteins are relatively more readily presented as antigens. The ease with which an antigenic protein is presented as an antigen can be evaluated using all of the measured values from the aforementioned three steps as indexes, or by using the measured values from any one or two of the steps as indexes.

The present invention also relates to methods for producing dendritic cells proficient at inducing CTLs. The present production methods comprise carrying out the above "methods for selecting proteins and such that are readily presented as antigens on dendritic cells", and collecting dendritic cells that have taken up a protein selected by these methods. Dendritic cells that have taken up proteins readily presented as antigens are thought to exhibit proficiency at inducing CTLs *via* peptides derived from these proteins and positioned on the surfaces of these dendritic cells.

The step of collecting dendritic cells may be carried out during the process of selecting proteins that are readily presented as antigens, or after protein selection is complete. An example is described. First, dendritic cells and a number of antigenic proteins are prepared. Dendritic cells are separately contacted with each of the antigenic proteins and cultured. Each of the dendritic cells contacted with an antigenic protein is subjected to each of the steps of measuring the amount taken up, measuring the polyubiquitination level, and measuring the residual amount, and these measurements are made using only a portion of the cultured cells. Proteins that are readily presented as antigens are selected based on the measured values for each step. From among the remaining cells, those dendritic cells cultured in the presence of the selected proteins are thought to be those dendritic cells that present peptides derived from "proteins that are readily presented as antigens" on the cell surface, and to exhibit proficiency at inducing CTLs; such cells can be used as is as "dendritic cells proficient at inducing CTLs". Alternatively, proteins that are readily presented as antigens can first be selected by the aforementioned "methods for selecting proteins and such that are readily presented as antigens on dendritic cells", and then dendritic cells are made to take up the selected proteins, and these dendritic cells can be collected.

The "methods for selecting proteins and such that are readily presented as antigens on dendritic cells" and the "methods for producing dendritic cells proficient at inducing CTLs" of the present invention are particularly useful in cancer immunotherapies. Dendritic cell vaccines that use the proteins and such selected by the methods of the present invention and that use the dendritic cells produced by the methods of the present invention are considered to strongly induce immunity against cancers, and improvements in therapeutic effects can be expected. Therefore, the "methods for selecting proteins and such that are readily presented as antigens on dendritic cells" and the "methods for producing dendritic cells proficient at inducing CTLs" of the present invention may be used to examine whether certain proteins or dendritic cells can be suitably used as vaccines that use cancer antigens. Furthermore, by carrying out the methods of the present invention for proteins derived from infectious microorganisms, it may be possible to produce dendritic cell vaccines for use in treating infections.

All prior art references cited herein are incorporated by reference into this description.

### Examples

The present invention is explained more specifically below with reference to Examples, but it is not to be construed as being limited thereto.

### [Example 1] Proteasome-dependent degradation of accumulated OVA

Intracellular localization of foreign antigens was examined by cell fractionation methods. Chicken ovalbumin (OVA) was used as an antigen model, and DC2.4 (haplotype H-2Kb), a cultured cell line of mouse dendritic cells, was used as a model for dendritic cells. The DC2.4 cell line is derived from dendritic cells, and was established while retaining many of the properties of dendritic cells, such as cross-presentation. BMDCs with a haplotype of H-2Kb, the same as that of DC2.4, were used as the control. BMDCs were induced from the bone marrow cells of C57BL/6 mice (SLC) in RPMI-1640 supplemented with 5% FCS using 5 ng/mL GM-CSF (MBL), and those cells purified by magnetic beads after five days of culturing were used.

DC2.4 cells made to take up bOVA antigens were suspended in 10 mM Tris-HCl (pH7.4) containing 250 mM sucrose, this was homogenized using glass beads, centrifuged for 30 minutes at 250,000 x g, and then fractionated. The supernatant was used as the soluble fraction; the precipitates were suspended in the same buffer as described above and used as the microsomal fraction. A fixed amount was incubated in the presence or absence of 1% TritonX-100, and together with or without 100 µg mL⁻¹ trypsin. Before collecting bOVA, soybean trypsin inhibitor (Sigma) was added at 500 µg mL⁻¹.

As a result of the above, 70% or more of bOVA taken up by cells was recovered as trypsin-resistant bOVA from the microsomal fraction. When bOVA collected in the microsomal fraction was treated with TritonX-100, it was degraded by trypsin. This suggests that most of the bOVA taken up was accumulated inside membranous subcellular structures (Fig. 1a). Further, BiP, an endogenous ER protein, was detected in the same microsomal fraction (Fig. 1a). Therefore, the microsomal fraction was found to comprise the ER fraction.

Next, DC2.4 cells were incubated with bOVA for one hour, and this was then chased for up to four hours. Cell-bound bOVA was recovered from total cell lysates by streptavidin-agarose and separated by SDS-PAGE. Cell-bound bOVA decreased with time, but this decrease was blocked in the presence of lactacystin or MG-132, which are proteasome inhibitors (Figs. 1b and 1c). Ammonium chloride, which is a lysosome inhibitor, did not significantly affect bOVA degradation (Fig. 1c). This suggests that lysosomal proteases are not involved in antigen degradation.

Essentially similar results were obtained from DC2.4 cells exposed to GST-OVA instead of bOVA and from bone marrow-derived DCs (BMDCs) exposed to bOVA (data not shown).

The majority of the cell-bound bOVA had the same molecular weight as untreated bOVA (Fig. 1d). In addition, a notable amount of bOVA was found to be polyubiquitinated, as shown by anti-polyubiquitin antibodies and anti-OVA antibodies (Fig. 1d). These results suggest that undegraded bOVA accumulates in membranous organelles and is degraded *via* the ubiquitin-proteasome pathway. Protein degradation with such characteristics is essentially the same as that known as ER-associated degradation (ERAD). The present inventors provide the following three lines of evidence indicating that bOVA accumulated in DC2.4 cells is degraded by ERAD, or if not identical, by a very similar protein degradation mechanism.

### [Example 2] Relationship between accumulated OVA and ERAD-related proteins

To prove the above-mentioned hypothesis, first, the relationship between accumulated OVA and substances involved in retrograde transport from the ER to the cytoplasm was examined by co-immunoprecipitation. 2x10⁷ DC2.4 or BMDC cells were cultured for four hours in a medium containing 10 µM MG-132 in the presence of bOVA at 250 µg mL⁻¹. Cells were lysed in 20 mM HEPES pH7.6 containing 1% digitonin and protease inhibitors to collect biotin-labeled samples. The samples were cleared of impurities in advance using protein G sepharose (Amersham Pharmacia Biotech), and after incubation with antibodies for precipitation (anti-Sec61β, anti Sec61α, anti-VCP, anti-BiP, anti-PDI, anti-calreticulin, anti-TAP1, and anti-TAP2), the immunoprecipitates were collected using protein G When chicken anti-CHIP antibody was used, immunoprecipitates were collected using a rabbit anti-IgY column (Mr. S. Seki, MBL). The precipitated samples were analyzed by SDS-PAGE and Western blotting.

When the Sec61 complex was immunoprecipitated with an anti-Sec61β antibody or anti-Sec61α antibody, a portion of bOVA was observed to be bound to Sec61 in DC2.4 cells incubated with bOVA in the presence or absence of lactacystin (Figs. 2a and 2b). Binding between bOVA and VCP was also indicated when a sample from DC2.4 cells incubated with bOVA was subjected to immunoprecipitation with an anti-VCP antibody (Fig. 2c). Three types of ER resident proteins - BiP, protein disulfide isomerase (PDI), and calreticulin - were also found to bind to OVA (Figs. 2d-f). Interestingly, a portion of bOVA was revealed to bind to CHIP, which is an E3-ubiquitin ligase (Fig. 2g). In contrast to the above-mentioned proteins, bOVA did not bind to TAP 1 or TAP2 (Figs. 2h-j).

To further confirm the above binding between the ERAD-related proteins and bOVA, copurification experiments were carried out. Simultaneous purification experiments were carried out as follows: DC2.4 cells and BMDCs were individually incubated with bOVA (250 µg mL⁻¹), washed twice with PBS, and then lysed in a digitonin buffer (1% digitonin in 20 mM HEPES pH7.6 for use in copurification) together with a protease inhibitor cocktail (Sigma). The samples were precipitated using streptavidin-agarose beads (SA beads) (Novagen) for one hour at room temperature. The precipitated samples were separated on 7.5-15% SDS-PAGE (Biocraft) and transferred onto Immobilon P (Millipore) for Western blotting. The protein bands were visualized by chemiluminescence methods (Amersham Pharmacia Biotech).

Binding of BiP, Sec61α, Sec61β, VCP, calreticulin, and CHIP with bOVA was also demonstrated by copurification, from the cell lysates, of bOVA and the above proteins using streptavidin beads (Fig. 3a). The absence of binding between TAP1/2 and bOVA was again observed in the present purification experiments. Bone marrow-derived DC (BMDC) lysates incubated with anti-Sec61 antibody or anti-VCP antibody immunoprecipitated with bOVA (Figs. 2j and 2k), and BiP and VCP were co-purified with bOVA.

It is worth noting that the majority of bOVA, in DC2.4 cells, that bound to the above molecules including CHIP had the same molecular weight as untreated bOVA (Figs. 2a-g). These results suggest that denatured bOVA accumulates in the ER or ER-related structures without being degraded, is transported to the cytoplasm *via* Sec61 transporters, then undergoes polyubiquitination which at least partly involves CHIP, and is finally subjected to degradation by proteasomes.

### [Example 3] Decrease caused by ERAD inhibition of the degradation of accumulated OVA

Next, to further confirm the involvement of ERAD, the effect on bOVA accumulation of ERAD inhibition, for example by Ca²⁺ depletion or thapsigargin treatment, was examined. Depletion of Ca²⁺ in the ER is known to inhibit ERAD. Thapsigargin (Tg) is known to decrease the Ca²⁺ concentration in the ER lumen by binding to the ER membrane and functioning as an ionophore and to inhibit ERAD. Thus, in the presence of Tg, bOVA degradation by ERAD is predicted to be suppressed. DC2.4 cells were pulsed with bOVA in the presence of MG-132, then washed with PBS or with PBS containing 1 mM EGTA, and then chased for four hours. As shown in Fig. 4a, accumulated bOVA did not decrease with Ca²⁺ depletion. Re-addition of Ca²⁺ to cells pre-exposed to EGTA restored bOVA degradation (Fig. 4a). This eliminates the possibility that Ca²⁺ depletion caused irreversible damage to DC2.4 cells. Thapsigargin also inhibited the degradation of accumulated bOVA (Fig. 4b).

Furthermore, the effect of tunicamycin treatment on bOVA accumulation was examined. When cells are treated with tunicamycin (Tm), N-type glycosylation is inhibited, and abnormally folded proteins accumulate in the ER lumen. Since such structurally abnormal proteins are good ERAD substrates, treatment by ERAD of bOVA taken up is predicted to be competitively inhibited by the misfolded proteins formed by tunicamycin treatment. When DC2.4 cells were treated with tunicamycin, as expected, bOVA degradation was inhibited (Fig. 4b). Tunicamycin treatment resulted in the accumulation of N-glycosylation-deficient proteins in the ER, which were good ERAD substrates and therefore competed with bOVA for degradation.

As the third evidence for the above hypothesis, RNAi methods were performed to knock-down proteins involved in the retrograde transport of substrates in the ERAD pathway (Fig. 4c). A 1.2 kb *Pvu*II fragment derived from pSilencer1.0-U6 (Ambion) was inserted into the *Sma*I site of pEGFP-C1 (BD Biosciences) to obtain p60. p600ΔC was produced by deleting the 1.3 Kb *Ase*I*-Bgl*II fragment from p60. siRNA sequences targeting Sec61, VCP, or CHIP were inserted into p60ΔC (Sec61-22), p60 (VCP-41), or the pSilencer2.1-U6hygro vector (Ambion) (CHIP-4) according to the manufacturer's protocol. Out of five target sequences for Sec61α, two were significantly effective for Sec61α knock-down. Three were not effective. Two of the five target sequences for VCP and one of the five target sequences for CHIP were significantly effective. The effective target sequences used in the present Examples are the following:
Sec61-22: AATGATCATTACTATCGGT (SEQ ID NO: 7);
VCP-41: AATCCTTGAATGAAGTAGGCT (SEQ ID NO: 8); and
CHIP-4: CAGTATCGAGGAACGGCGC (SEQ ID NO: 9).

In Sec61α-knocked-down DC2.4 cells (S22 and S22-3), accumulated OVA decreased more slowly than in the parental DC2.4 cells (Figs. 4d and 4e). Scrambled siRNA sequences for Sec61α knock-down did not affect the expression level of Sec61α nor the accumulation of bOVA (data not shown). These results suggest that Sec61α is involved in the transport to direct bOVA to degradation. Similarly, VCP-knocked-down DC2.4 cells (V41 and V41-1) retained larger amounts of bOVA than parental DC2.4 cells during chase (Figs. 4d and 4e), although accumulated bOVA decreased to some extent.

When CHIP-knocked-down DC2.4 cells (C4 and C4F-1) were produced to examine the polyubiquitination of bOVA when CHIP is knocked down, the uptake of bOVA into the CHIP-knocked-down DC2.4 cells was only about 70% that of uptake into the parental DC2.4 cells (Figs. 4d and 4f). Despite this fact, polyubiquitination of bOVA was reduced to 34% in CHIP-knocked-down cells (Fig. 4f). The decrease in the amount of accumulated OVA was slowed down in CHIP-knocked-down cells (Figs. 4d and 4e). In contrast, TAP1 knock-down did not affect bOVA degradation in DC2.4 cells (Fig. 5).

### [Example 4] Localization of accumulated OVA in DC2.4 cells

All of the above results strongly support that bOVA taken up by DC2.4 cells is degraded through ERAD. However, proteomic analyses and immunoelectron microscopic observations have recently revealed that phagosomes, which are where infectious bacteria are taken up, comprise a group of proteins that constitute the ER, including TAP1/2 and substances involved in antigen presentation, such as MHC. Accordingly, the present inventors decided to examine the intracellular localization of accumulated OVA in DC2.4 cells and BMDCs by using immunofluorescence confocal microscopy. DC2.4 cells and BMDCs on culture slides (FALCON) were pretreated for 30 minutes with MG-132, and then incubated for two hours with OVA at 500 µg mL⁻¹. After washing three times with the medium, the cells were fixed for ten minutes with 3.7% formaldehyde, permeabilized by treating with 0.1% TritonX-100 in PBS for ten minutes at room temperature, incubated with a designated antibody in PBS containing 5% BSA/2% FCS at 37°C for one hour, and stained. Alexa Fluor 488 goat anti-mouse IgG F(ab')₂ fragments, Alexa Fluor 488 goat anti-rat IgG, and Alexa Fluor 546 goat anti-rabbit F(ab')₂ fragments (Molecular Probes) were used as the secondary antibodies. Images were captured using an IX70 (Olympus) inverted microscope equipped with a fluorescence apparatus and processed using Deltavision deconvolution microscopy software (Applied Precision).

Spots or speckles of OVA were detected in the cells, but these OVA distributions did not overlap with either early endosomes (immunostaining of EEA1) or caveosomes (immunostaining of caveolin-1), which are known to be involved in the uptake of foreign substances (Figs. 6a and 6b). Instead, OVA immunofluorescence colocalized at least in part with late endosomes (stained with anti-Lamp1 antibody) (Fig. 6c) and ER (Fig. 6d). Although ER labeled with the anti-KDEL antibody showed a lace-like structure, OVA was not uniformly distributed throughout the ER (Fig. 6d). Similarly, OVA was detected in restricted areas of late endosomes (Fig. 6c). OVA did not colocalize with the Golgi apparatus (stained with anti-GM-130 antibody) (Fig. 6f). Localization of accumulated OVA in the ER was also confirmed with BMDCs (Fig. 6e). These results are consistent with the hypothesis of the present inventors that the OVA taken up into DC follows ERAD and/or a similar protein degradation pathway in other organelles comprising ER constituents, such as phagosomes and late endosomes.

### [Example 5] Subcellular fractionation of OVA-containing membrane vesicles by density-gradient centrifugation

Next, the present inventors examined the distribution of accumulated OVA in membrane fractions of DC2.4 cells by using iodixanol gradient centrifugation. For the density gradient centrifugation, DC2.4 cells incubated with bOVA (500 µg/mL) for three hours were washed twice with PBS, suspended in a homogenization medium (0.25 M sucrose, 1 mM EDTA, and 10 mM Hepes-NaOH pH7.4), and ruptured by ten strokes of a Dounce homogenizer. Unruptured cells and nuclei were removed by centrifugation at 2,000 x g for ten minutes. The nuclei-removed (postnuclear) supernatant was centrifuged at 100,000 x g for 45 minutes to obtain a pellet, this was resuspended in a homogenization medium and layered onto a 2.5-30% or 10-30% discontinuous Optiprep (Gibco/Invitrogen) gradient Centrifugation was carried out at 4°C with a Beckman SW 60Ti rotor, at 200,000 x g for 2.5 hours in the case of 2.5-30% gradient, and at 300,000 x g for three hours in the case of 10-30% gradient. Eleven fractions obtained from the top layer of each centrifuge tube were TCA precipitated, and then analyzed by SDS-PAGE and Western blotting.

Although OVA was detected in all the fractions, the peak of OVA was clearly observed in fraction #10 in the 2.5-30% density gradient (Fig. 7a) and in fraction #5 in the 10-30% density gradient (Fig. 7b). Distribution of Sec61, BiP (KDEL), and Rab5 in the fractions was similar to that of OVA (Fig. 7). In contrast, Lamp-1, a late endosome marker, showed two peaks - fractions #6 and #10 - in 2.5-30% density gradient. GM130, a Golgi marker, and transferrin receptor (Tfr), a recycling endosome marker, were collected separately. All of these results are consistent with those obtained by immunofluorescence microscopy.

### [Example 6] Reduction of cross-presentation due to knock-down of Sec61, VCP and CHIP

Whether knock-down of cellular components involved in ERAD decreases the cross-presentation of exogenous OVA antigen was examined using a system for evaluating the level of antigen presentation, which uses naive CD8⁺ T cells from OT-I mice and DC2.4 cells. The DC2.4 cells used as a model of dendritic cells are of the H-2Kb mouse MHC class I haplotype. OT-I (H-2Kb) mice are transgenic mice expressing a T cell receptor (TCRα, and β) that recognizes an antigenic peptide (SIINFEKL) (SEQ ID NO: 15) derived from OVA presented on the H-2Kb molecules. The CD8⁺ cells of OT-I mice are activated when they recognize OVA peptides presented by BMDCs derived from H-2Kb mice or by DC2.4 cells. When OVA or bOVA is incubated with and affixed onto DC2.4 cells and the cells are further incubated with naive CD8⁺ T cells of OT-I mice, the amount of IL-2 produced by OT-I CD8⁺ T cells serves as an index of T-cell stimulation as a result of OVA antigen presented by DC2.4 cells.

Parental or knocked-down DC2.4 cells were pre-incubated in a culture medium supplemented with 50 µg mL⁻¹ polymyxin B for 30 minutes in the presence or absence of lactacystin or MG-132, and incubated with OVA (250 µg mL⁻¹) or SIINFEKL (SEQ ID NO: 15) peptides (10 ng mL⁻¹) for six hours. The cells were washed, fixed with 1% paraformaldehyde, and aliquoted into microtiter plates (5 x 10⁴ per well). 5 x 10⁴ CD8⁺ T cells purified from splenocytes of OT-1 mice using magnetic beads were added to each well and after a 24-hour incubation, the IL-2 secretion was measured by ELISA (BD Biosciences) in triplicate.

As shown in Fig. 8a, OVA and bOVA were equally antigenic for stimulation of CD8+ OT-I T cells. As expected, proteasome inhibitors suppressed OVA antigen presentation (Fig. 8b). In contrast, NH₄Cl did not reduce the presentation of the OVA antigen with MHC class to OT-I CD8⁺ T cells, but inhibited the presentation with MHC class II to OT-II CD4⁺ cells (Fig. 9). The effect of knocking down Sec61, VCP, or CHIP was examined using uncloned DC2.4 transfectants into which siRNA expression vectors had been introduced (S22, V41, and C4), as well as cloned transfectants (S22-3, V41-1, and C4-1) (Figs. 8c and 8d). Since Sec61 is involved in both forward and retrograde transport of ER-associated proteins, it was not surprising that the knock-down of Sec61 reduced antigen presentation of OVA added from outside the cell. Knock-down of VCP necessary for the retrograde transport of ER-associated proteins also reduced antigen presentation by DC2.4 cells. These results support the hypothesis of the present inventors that exogenously added OVA is processed for cross-presentation *via* ERAD. Furthermore, in accordance with the effect of OVA accumulation in DC2.4 cells, knock-down of CHIP resulted in a decrease in OVA cross-presentation (Figs. 8c and 8d), suggesting that CHIP functions as an E3-ligase for OVA polyubiquitination during cross-presentation.

### [Example 7] Promotion of polyubiquitination by addition of an endoplasmic reticulum localization sequence

There are reports that when the KDEL sequence (Lys-Asp-Glu-Leu) (SEQ ID NO: 3), an endoplasmic reticulum localization sequence, is added to the carboxyl terminus of a protein localized in the ER lumen, the protein is repeatedly transported between the ER and Golgi apparatus, and ERAD of that protein is promoted. Therefore, DC2.4 cells were made to take up OVA with KDEL (SEQ ID NO: 3) added to its C-terminus, and OVA that was polyubiquitinated after uptake was observed by SDS-PAGE and Western blotting. OVA to which a KDEL sequence was added was constructed and expressed as follows: using a plasmid comprising a DNA sequence encoding OVA as a template, PCR was performed using oligo-1 (SEQ ID NO: 10) and oligo-2 (SEQ ID NO: 11) as primers. The amplified PCR product of approximately 1100 bp was introduced into a pCR-Blunt vector (Invitrogen) to obtain vector-1. Oligo-3 (SEQ ID NO: 12) and oligo-4 (SEQ ID NO: 13) were annealed, and the obtained gene fragment of approximately 100 bp was introduced into the pCR-Blunt vector to obtain vector-2. Vector-2 was digested with *Spe*I*,* the obtained gene fragment of approximately 130 bp was introduced into vector-1 that had been digested with *Nhe*I to obtain vector-3. An approximately 1250-bp gene fragment (OVA-KDEL) obtained when vector-3 was digested with *BamH*I/*Spe*I was introduced into a vector for gene expression, and then expressed. The amino acid sequence of OVA to which a KDEL sequence has been added is shown in SEQ ID NO: 14.

The results are shown in Fig. 10. Compared to when DC2.4 cells were made to take up OVA, uptake of OVA which has a KDEL (SEQ ID NO: 3) added to its C-terminus resulted in significantly promoted polyubiquitination of the substrate (OVA-KDEL).

Moreover, (Lys-Asp-Glu-Leu) (SEQ ID NO: 3) was added to the carboxyl terminus of OVA, DC2.4 cells were made to take this up, and the antigen presentation levels of OVA and OVA-KDEL were observed using the aforementioned antigen presentation level evaluation system that uses OT-I mouse CD8⁺ T cells. Antigen presentation was clearly found to be stronger with OVA-KDEL than with OVA (data not shown).

The above results showed that when OVA and OVA-KDEL are compared, OVA-KDEL is more readily polyubiquitinated, and more efficiently presented as antigen. Therefore, a correlation was found between ease of polyubiquitination and ease of presentation as an antigen. Therefore, the intensity of cross-presentation of an antigenic protein can be estimated from the results of measuring the polyubiquitination of the antigenic protein.

When OVA is the external antigen, as described above, an antigen presentation assay using OT-I mouse cells can be constructed. However, in general, animals that express TCR specific to a particular antigen do not exist, therefore, construction of an antigen presentation assay such as that with OVA is impossible. Therefore, when there is a need to know how readily a certain antigen is presented, it is very useful to estimate the ease of antigen presentation by measuring the uptake of the antigenic protein into dendritic cells and the polyubiquitination of the antigenic protein.

### [Example 8] Promotion of polyubiquitination by Hsp

DC2.4 cells were incubated with OVA (0, 50, or 250 µg/mL) in RPMI1640/10% FCS for six hours at 37°C, in the presence or absence of pig-derived HSP90 (10 µg/mL). After washing away the antigen and HSP, OT-I CD8⁺ T cells were added, cells were cultured overnight at 37°C, and the amount of IL-2 comprised in the supernatant was measured by the ELISA method. As a result, a stronger antigen presentation was observed than when incubation was carried out with OVA alone (Fig. 11-(a)). Moreover, the antigen presentation ability increased in an Hsp90 concentration-dependent manner (Fig. 11-(b)).

Next, DC2.4 cells were incubated for a set amount of time with OVA in the presence or absence of lactacystin (LC), and OVA taken up into the cells was measured. OVA taken up into cells in the presence or absence of Hsp90 was measured similarly. DC2.4 cells were observed to take up OVA into cells more efficiently in the presence of Hsp90 than in the absence of Hsp90 (Fig. 12).

DC2.4 cells were incubated with GST-OVA in the presence or absence of Hsp90, cells were collected, and an extract solution was produced. After subjecting this to SDS-PAGE, Western blotting was performed using an anti-polyubiquitin antibody. OVA taken up into DC2.4 cells were shown to be more strongly polyubiquitinated in the presence of Hsp90 than in the absence of Hsp90 (Fig. 10).

According to the above results, compared to incubation with OVA alone, incubation with OVA in the presence of Hsp90 increases the amount of OVA taken up by cells, and the amount of polyubiquitination was also found to increase. It can be concluded from this finding that measuring the antigen presentation of a certain antigenic protein can be substituted with measuring the uptake of an antigenic protein into dendritic cells and the polyubiquitination of the antigenic protein.

### Industrial Applicability

The present invention provided generally applicable methods for selecting antigenic proteins that are readily presented as antigens. In cancer immunotherapy, selecting cancer-specific proteins to use as vaccines for cancer therapy is an important process that influences subsequent therapeutic effects. If antigenic proteins that are readily presented as antigens can be pre-selected, it may be possible to provide more suitable treatments in cancer immunotherapies. This paves the way for selection of the most suitable proteins from among the many cancer-specific proteins that are candidates for cancer therapy vaccines, at the level of the individual patient, and thus it may enable so-called "tailor-made" therapies. Therefore, the methods of the present invention may improve the therapeutic effects of cancer immunotherapies through selection of more suitable cancer-specific proteins.

## Claims

1. A method for selecting a protein that is readily presented as an antigen on a dendritic cell, wherein a step of contacting a protein to an isolated dendritic cell is followed by at least one or more of the following steps of:
(a) measuring the amount of the protein taken up in the dendritic cell;
(b) measuring the amount of the protein polyubiquitinated in the dendritic cell; and
(c) measuring the amount of the protein taken up by the dendritic cell that remains in the dendritic cell.

2. A method for producing a dendritic cell proficient at inducing a CTL, wherein the method comprises the steps of:
contacting a protein to an isolated dendritic cell;
measuring at least any one or more of the following amounts of protein:
(a) the amount of the protein taken up in the dendritic cell;
(b) the amount of the protein polyubiquitinated in the dendritic cell; and
(c) the amount of the protein taken up by the dendritic cell that remains in the dendritic cell;
selecting a dendritic cell that has taken up the protein, based on the measurements in the previous step; and
collecting the dendritic cell selected in the selection step.

3. The method of claim 1 or 2, wherein the protein is labeled and then used.

4. The method of any one of claims 1 to 3, wherein the step of measuring the amount of protein polyubiquitinated in the dendritic cell comprises the use of an anti-ubiquitin antibody.

5. A method for testing capacity as a cancer antigen vaccine, which comprises the method of any one of claims 1, 3, and 4.
